# EUROPEAN PATENT APPLICATION

(11) **EP 2 258 217 A1**
(43) Date of publication of application: **08.12.2010**
(21) Application number: 09161668.0
(22) Date of filing: 02.06.2009
(51) Int. Cl.: A23L 1/29, A23L 1/30

(54) **Nutritional Composition for Supporting Brain Development and Function of Children**

(71) Applicant: Nestec S.A., 1800 Vevey (CH)
(72) Inventor: Fleith, Mathilde, CH-1806 Saint-Legier (CH); Fukushima, Yoichi, Saitama 331-0805, (JP); Rapinett, Gertrude, CH-1096 Cully (CH); Schmitt, Jeroen Antonius Johannes, CH-1510 Moudon, (CH); Mateus, Maria-Luiza, CH-1052 Le Mont-sur-Lausanne (CH)
(74) Representative: Chautard, Cécile

(57) **Abstract**

The present invention relates to a nutritional composition, in particular directed to children of 3-6 years, said nutritional composition comprising a protein source, a source of available carbohydrates, a lipid source, at least one probiotic microorganism, and prebiotics, wherein said lipid source comprises DHA (docosahexaenoic acid) and/or ARA (arachidonic acid). The nutritional composition improves cognitive performance, in particular memory, learning comprehension, alertness, attention, concentration, processing speed, conceptual thinking, abstract thinking, verbal abilities, language comprehension, psychomotor skills, curiosity, and confident interaction with the environment. Preferably, the composition comprises one, a combination of several or all selected of the group of DHA, ARA, LA, ALA, choline, iron, iodine and folic acid.

## Description

### Technical Field

The present invention relates to a nutritional composition. More particularly, the present invention relates to a nutritional composition for improving the cognitive performance of children, in particular children of an age of 3 to 6 years. The present invention further relates to a method for supporting and/or improving cognitive performance in a child of 3 to 6 years, to providing nutrition, the method comprising the step of administering to a child of 3 to 6 years the nutritional composition of the present invention, and a method for preparing a nutritional composition useful for supporting and/or improving cognitive performance.

### Prior Art and the Problem Underlying the Invention

Breast milk is the right milk for infants up to the age of 12 months. In some situations, breast milk is not available or not the preferred option of providing nutrition to an infant, and infant formulas, generally based on cow's milk, have been developed for these situations. In the period starting from the age of about 7 to 18 months, a child is generally weaning and starts to take up solid food. Instead of milk-based infant formula, pure milk is generally used to provide important nutrients of milk to the child at this age.

However, at the age starting from about one year and up to about 6-7 years, cow's milk alone is not suitable to meet the increased needs of some nutrients, such as iron, for example. Growing up milks have been developed to provide a more balanced and complete food, better adapted to the child's nutritional needs at this age, than normal milk. Accordingly, the objective of follow-up formulae and growing-up milks has generally been to provide nutrients that are important for supporting the healthy growth of a child.

More recently, it has also become an objective to strengthen a child's immune defences. For example, in US2007/0031537, a formula comprising LC-PUFA (long-chain poly unsaturated fatty acids) and a probiotic is disclosed. However, the formula disclosed in this document is directed to infants, not to children above 1 year.

In addition to strengthening a child's immune defences and improving a child's health, physical well-being and mood, the present invention addresses the problem of providing further benefits to children of the age of about 3 to 6, preferably 3 to 5 years.

In addition, the present invention has to objective to provide a nutritional composition for children of the indicated age, said nutritional composition supports normal brain and/or mental development of the child and/or mental and/or cognitive performance.

Furthermore, the present invention has to objective to provide a nutritional composition that contributes to a good performance of a child in school, to an adaptive behaviour, to cognitive skills, language skills, motor skills and socio-emotional skills.

For example, the present invention addresses the problem of providing nutritional composition that contributes to or even improves the normal cognitive, behavioural, emotional, language and intellectual development of a child.

The present invention also addresses the problem of providing a nutritional composition comprising nutrients that are important for the development of structural components of a child's brain. In other words the invention provides a nutritional composition providing the right nutrition for the developing brain of a child. This implies nutrients that the child's body needs for building up the brain ("structural building blocks"), nutrients that are necessary for the processes, such as metabolic processes, taking place in the brain, and nutrients that provide an adequate availability of energy that the brain needs for proper functioning.

More specifically, the present invention addresses the problem of providing a nutritional composition that contributes to or even improves cognitive capacities and properties such as short and long term memory, learning capacity, comprehension, planning and reasoning, psychomotor skills, attention and concentration capacity of a child.

It is important to note that the present invention addresses the above problems while at the same time providing a balanced nutrition, providing important macro- and micronutrients, such as proteins, carbohydrates and fats, and vitamins and trace elements, respectively.

The present invention addresses the problems depicted above, which problems thus are integral part of this invention.

### Summary of Invention

The present invention provides a nutritional composition comprising a protein source, a source of available carbohydrates, and a lipid source, wherein said lipid source comprises DHA (docosahexaenoic acid).

In another aspect, the present invention provides a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

According to an aspect, DHA is provided in the form of an oil rich in LC-PUFA (long-chain polyunsaturated fatty acids).

According to an aspect, the nutritional composition of the invention is used to support and/or improve cognitive performance in a child of 3 to 6 years.

According to an aspect, the invention provides a method for one or more selected from supporting, sustaining and improving cognitive performance in a child of 3 to 6 years, said method comprising the step of administrating a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

According to an aspect, the invention provides a method of providing nutrition, the method comprising the step of administering to a child of 3 to 6 years a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

According to an aspect, the invention provides a method for preparing a nutritional composition useful for supporting and/or improving brain function, said method comprising mixing:
- one or more protein source,
- one or more source of available carbohydrates,
- one or more lipid source,
- one or more probiotic microorganism, and,
- prebiotics,
wherein said lipid source comprises DHA (docosahexaenoic acid), and thereby obtaining the nutritional composition of the invention.

According to an aspect, the present invention provides the use of a protein source, a source of available carbohydrates, a lipid source and other ingredients as disclosed in this specification for preparing a nutritional composition.

Remarkably, the nutritional composition as defined by the present invention and its preferred embodiments, if used as a nutritional complement in children of 3 to 6, preferably 3 to 5 years, provides nutrients useful to contribute to the development of the brain and to its proper functioning.

Furthermore, the nutritional composition is useful as a growing up milk, contributes to or even improves the cognitive, behavioural, emotional, language and intellectual development of a child.

Accordingly, the present invention provides, in an aspect, the use of the nutritional composition as disclosed herein for supporting and/or improving the verbal, perceptual, quantitative abilities, as well as memory development in a child of 3 to 6, preferably 3 to 5 years of age.

In a similar aspect, the present invention provides, in another aspect, the use of the nutritional composition as disclosed herein for supporting and/or improving behavioural, cognitive, communicational, such as language, expressive and receptive communication, and social-emotional skills in a child of 3 to 6, preferably 3 to 5 years of age.

Further, aspects and preferred embodiments of the present invention are provided in the description below as well as in the appended claims.

### Detailed Description of the Preferred Embodiments

The nutritional composition preferably comprises a protein source, a source of available carbohydrates, a lipid source, at least one probiotic microorganism, and, preferably, prebiotics. The term "comprises", for the purpose of the present invention means "includes, amongst other" and is not intended to mean "consists only of".

In the present specification, the term "nutrient" is repeatedly used. The term "nutrient" refers to typical macro-and micronutrients, such as proteins, lipids, carbohydrates, vitamins and trace elements, for example. However, for the purpose of the present specification, the term nutrient may also be used with respect to ingredients that are added for the purpose of exerting any kind of beneficial purpose on the child, for example, non-essential but beneficial components such as, for example, probiotics, fiber, in particular prebiotics, DHA (docosahexaenoic acid) and other functional ingredients with beneficial effects on a child's development.

The nutritional composition of the invention preferably comprises all macronutrients, that is, proteinogenic matter (a protein source), lipids and available carbohydrates. According to an embodiment, said protein source provides about 10-22 percent of the total energy, said source of available carbohydrates provides from about 30-60 percent of the total energy, and said lipid source provides from about 30-55 percent of the total energy of the composition. More preferably, said protein source provides about 11-18 percent, said source of available carbohydrates provides from about 35 - 55 percent, and said lipid source provides from about 30-50 percent of the total energy of the composition. Most preferably, the protein source provides 12-16 percent, the carbohydrate source 40-50 percent, and the lipid source 35-45% of the total energy of the composition. Further information of quantities of macro- and micronutrients are given further below.

The protein source may be of vegetal or animal origin, for example. Examples of protein sources are soy protein, rice protein, wheat protein, milk protein and egg protein. Preferably, the nutritional composition comprises milk protein. Preferably, the composition comprises whey protein, casein, or both. More preferably, the composition comprises whey protein and casein. The protein source may be provided in the form of intact protein, partial and total protein hydrolysates, di-, tri-, oligo- and polypeptides, free amino acids, and mixtures of the afore-mentioned, for example. Free amino acids may, for example, be added to intact protein and/or a protein hydrolysate in order to provide a balanced amino acid profile.

Milk, for the purpose of the present invention, is generally obtained from domesticated animals, such as cow, goat, sheep, camel, dromedary, buffalo, and so forth. Preferably, milk is cow's or goat's milk. Accordingly, milk protein may be, for example, cow's milk protein. Milk contains several nutrients that are implied in the development and the functioning of the brain, thereby supporting cognitive development of a child. Without wishing to be bound by theory, the present inventors believe that by combining the ingredients naturally contained and delivered by milk, if combined with further nutrient in a nutritional composition, mutually interact and together synergistically affect brain development and functions as defined in greater detail further below.

The expression "available carbohydrates" refers to carbohydrates that break down during digestion, in particular in the upper digestive tract during the cephalic and intestinal phase, under the action of digestive enzymes produced by the human body, but also to carbohydrates that are directly absorbed, such as glucose. In particular, the available carbohydrates refer to a source of carbohydrate such as sugar (for example saccharose, glucose, fructose) and starch that can be digested by human enzymes. Generally, the available carbohydrates are absorbed and enter into the intermediary metabolism. Generally, available carbohydrates, if not directly provided in the form of glucose, are carbohydrates, which can be converted by the body to glucose, since the latter is the energy source used by the brain under normal physiological conditions.

Preferably, available carbohydrates are provided in the form of at least one selected from lactose, saccharose, and maltodextrin, and preferably a combination of at least two or of all three of the latter. Preferably, lactose provides 30%-95%, more preferably 50-85%, most preferably 60-78% by dry weight of all available carbohydrates. Preferably, sugars provide 0-40%, more preferably 2-35%, most preferably 5-25% by dry weight of all available carbohydrates. Preferably, maltodextrin provides at least 0-60%%, more preferably 1-30%, most preferably 3-15% by dry weight of all available carbohydrates in the nutritional composition of the invention.

The lipid source includes lipids useful form human nutrition of any origin, such as vegetal, animal and/or fish oils or fat, for example. Lipid sources include lipids obtained from milk, eggs, seeds, nuts, fungi, microorganisms such as yeast and bacteria, for example. Lipid sources include sources containing phospholipids, mono- di-, triglycerides, fatty acids, sterol-related compounds such as cholesterol, sphingosines, ceramides, glycosphingolipids, gangliosides and the like. Preferred sources of lipids are milk, vegetal oils and eggs. For example, the nutritional composition comprises milk fat, vegetal oil and fish oil. The fish oil may be replaced, for example, by another oil rich in PUFAs, in particular those mentioned herein, such as DHA, ARA, and also preferably ALA and LA.

Among the lipids, there are important elements that the human body uses in order to develop the brain, especially during infancy and childhood. Nervous tissue has the second highest concentration of fatty acids after adipose tissue. Fatty acids constitute slightly more than 50% of the dry weight of the brain. Levels of long chain polyunsaturated fatty acids are particularly high in the cerebral cortex and the retina. Brain phospholipids contain a high proportion of arachidonic acid (ARA, 20:4 ω-6). Brain is also unusually rich in ω-3 fatty acids, in particular docosahexaenoic acid (DHA, 22:6 ω-3, also referred to as cervonic acid). DHA can account for up to 50% of phospholipid fatty acid in the cerebral cortex and the retina, being particularly abundant in the rod photoreceptors and in gray matter. This suggests that DHA is heavily involved in neuronal and visual functions. In the brain, DHA and ARA can be synthesised from essential fatty acids, such as linoleic acid (LA: 18:2 ω-6) and α-linolenic acid (ALA: 18:3 ω-3). In summary, DHA is a key component of cell membranes in the brain and the retina, where it accumulates during development.

The brain accumulates DHA during childhood, so that by the age of 5-6 years, most of the brain's physical growth is completed. It is thus important to provide lipids in a way that supports the brain development during this period.

According to an embodiment, the nutritional composition of the present invention comprises DHA. Preferaby, the nutritional composition comprises 10-60 mg (milligram) DHA per 100 g of the dry weight of the nutritional composition. More preferably, the nutritional composition comprises 15-50, and most preferably at least 20-40 mg DHA per 100 g of the dry weight of the nutritional composition.

Expressed per 100 kcal of nutritional formula, the nutritional composition preferably comprises about 10-60 mg, more preferably about 15-50, even more preferably 20-45, and most preferably 30-40 mg DHA per 100 kcal of the nutritional composition.

According to an embodiment, the nutritional composition comprises ARA. Preferaby, the nutritional composition comprises at least 10-60 mg ARA per 100 g of the dry weight of the nutritional composition. More preferably, the nutritional composition comprises 15-50, and most preferably 20-45, and most preferably 30-40 mg ARA per 100 g of the dry weight of the nutritional composition.

Expressed per 100 kcal of nutritional formula, the nutritional composition comprises about 2-15 mg, more preferably about 3-11, even more preferably 4-8, and most preferably 5-7 mg ARA per 100 kcal of the nutritional composition.

Preferably, the ration by weight of DHA : ARA is in the range from 4:1 to about 1:4, more preferably from 2:1 to about 1:2, even more preferably from about 1.5:1 to 1:1.5 and most preferably 1.2:1 1 to 1:1.2. A ratio close to 1:1 is particularly preferred.

The nutritional composition of the present invention preferably comprises linoleic acid (LA: 18:2 ω-6), α-linolenic acid (ALA: 18:3 ω-3) or preferably both. LA and ALA are both essential long chain polyunsaturated fatty acids (LC-PUFAs). LA may be transformed into ARA, and ALA into DHA by the body.

Accordingly, the nutritional composition preferably comprises 1-10 g, more preferably 2-8, even more preferably 3-7 g and most preferably 3.5-6 g of linoleic acid per 100 g of the dry weight of the nutritional composition.

According to an embodiment, the nutritional composition preferably comprises 200 mg - 1100 mg, more preferably 300-1000, even more preferably 400-800 and most preferably 500-700 mg, for example about 600 mg of α-linolenic acid per 100 g of the dry weight of the nutritional composition.

Preferably, the ratio of linoleic acid to α-linolenic acid in the nutritional composition of the invention is in the range (including endpoints) of 2:1 to 12:1, preferably 4:1 to 10:1, more preferably 5:1 1 to 8:1.

According to an embodiment, the ratio of ω-6 to ω-3 fatty acids in the nutritional composition of the present invention is 3:1 and 12:1, more preferably between 4:1 and 10:1, even more preferably 5:1 and 9:1. Preferably the ratio of ω-6 to ω-3 fatty acids is at least 4.5, more preferably at least 5. The human body cannot convert fatty acids from ω-6 to ω-3 and *vice versa.* Furthermore, linoleic acid and α-linolenic acid compete for the same enzymes of the metabolism, and they both inhibit each other's desaturation and elongation. Therefore, it is important to provide a balanced ratio of these essential fatty acids in the diet to ensure optimal growth and development.

ALA and DHA may be added in the form of oils, in particular triglycerides, containing substantial amopunts (for example, more than 5wt.%, more preferably more than 10wt.%) of ARA and/or DHA. Examples of such oils are fish oils or oils produced by microorganisms, such as phytoplankton, algae, fungi or bacteria. LA and ALA may be provided in the form of vegetal oils or fats, that is, oils obtained from plants, for example, in particular seeds and the like.

According to an embodiment, the nutritional composition comprises iron. Generally, iron is an essential component of several cellular functions such as the transport of oxygen, mitochondrial electron transport and DNA synthesis. Brain tissue is overall rich in iron. Iron is also involved in the production of myelin, in the synthesis of neurotransmitters and their breakdown. For example, iron is essential for the synthesis of dopamine, an important neurotransmitter for attention, executive functioning and motivation. Iron has been implicated in the organization of axonal growth and synaptogenesis. Today, iron deficiency is the most common single nutrient deficiency in the world. Associations between iron deficiency anaemia and deficit in cognitive or behavioural performance in children have been observed. Iron is thus very important for brain development.

Iron, if added as a nutritional supplement to a nutritional composition, is not always easily absorbed in the digestive tract. There are forms of iron that have low bioavailability. Therefore, according to an embodiment, iron is added in a form having high bioavailability. Iron can be added in the form of ferrous or ferric iron (II+ or III+ oxidation state). An example of ferric iron is iron pyrophosphate. According to a preferred embodiment, iron is added as added ferrous sulphate, including hydrates of ferrous sulphate.

According to an embodiment, the nutritional composition of the present invention comprises 2-50 mg, more preferably 4-30 mg, even more preferably 5-20 mg and most preferably 6-15 mg of iron per 100 g of dry weight of the nutritional composition.

According to an embodiment, the nutritional composition comprises choline. The human brain is rich in choline-containing compounds. Choline is present in gray matter (10% of dry weight), white matter (8% of dry weight) and is a component of myelin (12% of dry weight). According to an embodiment, the nutritional composition of the invention comprises at least 30 mg of choline, preferably 50-300 mg, more preferably 80-250, most preferably 110-190 choline per 100 g of the dry weight of the nutritional composition.

According to an embodiment, the nutritional composition of the present invention comprises zinc. Zinc (Zn) is an essential trace mineral that needs to be provided by the diet. Zinc is required for the biological function of more than 300 enzymes and stabilizes the tertiary structure of some proteins. In the central nervous system, zinc is concentrated in the synaptic vesicles of specific glutaminergic neurons, which are found primarily in the forebrain and connect with other cerebral structures. Furthermore, in the synapse, zinc appears to be involved in neurotransmission.

Zinc is present in sat least some milk components, such as milk and buttermilk, and small amounts may be added in this form to the nutritional composition. Preferably, zinc is added in sufficient amounts as a separate trace element, for example in the form of zinc sulfate. According to an embodiment, the nutritional composition of the present invention comprises at least 5 mg zinc per 100 g of dry weight of the nutritional composition. According to an embodiment, the nutritional composition comprises 5-50, preferably 7-40, more preferably 8-20 mg of zinc per 100 g of dry weight of the nutritional composition.

According to an embodiment, the nutritional composition comprises vitamin D. There is biological evidence to suggest an important role for vitamin D in brain development and function. Therefore, the nutritional composition preferably comprises at least 2 µg (microgram) vitamin D per 100 g of dry weight of the nutritional composition. According to an embodiment, the nutritional composition comprises 3-12, more preferably 4-10, most preferably 5-8 µg vitamin D per 100 g of dry weight of the nutritional composition.

According to an embodiment, the nutritional composition comprises thiamine. Thiamin, also known as vitamin B₁, is a water soluble vitamin of the B vitamin family. Thiamin functions as part of a coenzyme in energy-yielding systems, especially those involved in the metabolism of carbohydrates and to the breakdown of glucose to energy. Thiamin also plays a role in the conduction of nerve impulses. Studies showed that deficiencies in thiamine may lead to decrease of short term memory, confusion and irritability (behavioral problems), besides negative effects on muscles and the cardiovascular system. Therefore, the nutritional composition preferably comprises 0.2-2, more preferably at least 0.3-1.8, even more preferably 0.4-1.6 and most preferably 0.5-1.4 mg thiamin per 100 g of dry weight of the nutritional composition.

According to an embodiment, the nutritional composition comprises folic acid. Folic acid, also known as vitamin B₉, is required for cell division and maintenance, formation of DNA and RNA, and the synthesis and breakdown of amino acids. Folic acid acts as a coenzyme in the transfer of one-carbon units. In infants and children, folate deficiency can slow overall growth. It is assumed, for the purpose of the present specification, that folate up-take is associated with cognitive performance, such as memory performance. According to an embodiment, the nutritional composition of the present invention comprises at least 80 µg folic acid per 100 g of dry weight of the nutritional composition. According to an embodiment, the nutritional composition comprises 90-300 µg folic acid, preferably 100-250, more preferably 120-200 and most preferably 130-150 µg folic acid per 100 g of dry weight of the nutritional composition.

According to an embodiment, the nutritional composition comprises iodine. Iodine is a trace element that is necessary for normal production of thyroid hormones, normal growth and normal brain development. Studies show that iodine deficiency may lead to mental impairment such as manifested by poor school performance, reduced intellectual ability and impaired work capacity. In certain geographical areas, soil and water is naturally low in iodine, raising the risk of deficiencies in the population living in these areas. According to an embodiment, the present nutritional composition comprises at least 10 mg iodine, for example preferably 10-300 mg, preferably 15-270 mg, more preferably 20-250 mg, and most preferably 40-230 mg of iodine per 100 g of dry weight of the nutritional composition.

According to an embodiment, the nutritional composition comprises sphingomyelin. Sphingomyelins are a subgroup of sphingophospholipids, which in turn are part of the larger group of sphingolipids in general. Sphingomyelins consist of a ceramide moiety (for example sphingosine) and a phosphacholine as a polar head group. Sphingomyelin is a structural component of cellular membranes and is found in the membranes of neurons and glia cells, and also in axons. Sphingomyelin facilitates the efficient and rapid propagation of nerve electrical signals. In particular, sphingomyelin is a component of the myelin sheath surrounding the nerve cell axons. In the present nutritional composition, sphingomyelin is preferably provided as a natural component of milk, in particular cow's milk, the latter being preferably used for the preparation of the composition. The nutritional composition of the invention preferably comprises at least 20 mg, for example 20-220, preferably 25-200, more preferably 30-150 mg and most preferably 40-100 mg sphingomyelin per 100 g of the dry weight of the nutritional composition.

According to an embodiment, the nutritional composition comprises sialic acid. Sialic acids are a family of over 50 members of 9-carbon sugars, all of which are derivatives of neuraminic acid. The predominant sialic acid family found in humans is from the N-acetylneuraminic acid sub-family. Sialic acids are contained in milk, such as cow's and goat's milk, for example. In mammals, neuronal cell membranes have the highest concentration of sialic acid compared to the other body cell membranes. The majority of sialic acid is present in gangliosides. Gangliosides are cell membrane components consisting of a lipid portion and a carbohydrate portion containing at least one or more sialic acid residues. Sialic acid plays an essential role in the transmission and storage of information in the brain. In the present nutritional composition, sialic acid may be added separately, but is preferably provided in sufficient amounts by way of milk used as an ingredient of the nutritional composition. Preferably, the nutritional composition comprises at least 50 mg of sialic acid per 100 g of dry weight of the nutritional composition. According to an embodiment, the nutritional composition comprises at least 50 mg of sialic acid per 100 g of dry weight of the nutritional composition. According to a preferred embodiment, the nutritional composition comprises 60-250, preferably 70-150, more preferably 80-130 mg of sialic acid per 100 g of dry weight of the nutritional composition.

With respect to the nutrients iodine, sphingomyelin and sialic acid, these nutrients are not specifically added to the nutritional composition, but are present due to their presence in milk components, in particular, whole milk, skimmed milk and/or buttermilk, for example. Without wishing to be bound by theory, the inventors believe that the presence of some or all of these nutrients is relevant for the benefits reported for the nutritional composition disclosed herein. While the mere presence of this nutrients is considered relevant, the exact amounts are subject to fluctuations, which are dependent on the origin of the milk in terms of the animal from which it is harvested (cow, goat, etc), from the geographical origin, and also from the season. The values given herein are thus approximations and may be considered as medium to long term averages. Choline is also present in substantial amounts in milk components, but is also separately added.

According to an embodiment, the nutritional composition comprises at least one probiotic. Probiotics are living microorganisms which, when administered in adequate amounts confer a health benefit on the host. According to an embodiment, the nutritional composition comprises at least two different probiotic microorganisms. Preferably, each of said microorganisms is provided at about 1x10⁵ to 1x10¹² CFU per g of dry matter of the composition, preferably at least 5x10⁵ to 1x10⁹, more preferably at least 1x10⁶. The "x" means "times" and thus refers to a multiplication of the respective numbers. Preferably, a combination of two or more different probiotic microorganisms is selected that provides a health benefit, in particular when administered to children of one year and older, in particular children of the age classes as defined for the nutritional composition herein. Preferably, the two different probiotic microorganisms are selected from different species, more preferably from of two different genera. According to an embodiment, the nutritional composition comprises the at least two probiotic microorganisms, one of which of the genus *Bifidobacterium* and the other of the genus *Lactobacillus.* Preferably, the composition comprises one or more *Bifidobacterium* strains, which is selected from a *Bifidobacterium longum* and a *Bifidobacterium lactis* strain and a *Lactobacillus paracasei* strain. According to a preferred embodiment, the nutritional composition comprises the *Bifidobacterium longum* strain Bb536, deposited as ATCC BAA-999, and the *Lactobacillus paracasei* strain deposited as CNCM I-2116.

According to an embodiment, the nutritional composition comprises dietary fibers. Preferably, the composition comprises soluble fibers. According to an embodiment, the composition of the present invention comprises at least one prebiotic, for example fructooligosaccharides (FOS) and/or galactooligosaccharides (GOS). Preferably, the nutritional comprises one or more selected from inulin, FOS, or a mixture of inulin and fructooligosaccharides. Preferably, the nutritional composition comprises at least 1, more preferably at least 2 and even more preferably at least 3 g soluble fiber per 100 g of dry weight of the nutritional composition. According to an embodiment, the nutritional composition comprises 1 to 10 g, preferably 2 to 7 g, more preferably 3 to 4 g of soluble fiber. Preferably, the soluble fiber is a mixture of inulin and FOS, said mixture comprising at least 50%, more preferably at least 60% by weight of dry matter of FOS.

According to a preferred embodiment, the composition of the present invention comprises one, a combination of several or all nutrients selected from the group of DHA, ARA, LA, ALA, and iron. Preferably, the nutritional composition also comprises iodine. Preferably, these nutrients are provided in the amounts as detailed above, or elsewhere within this specification, for example in the tables below.

According to an embodiment, the composition of the invention further comprises one, a combination of several or all nutrients selected from the group sphingomyelin and sialic acid. Preferably, the nutritional composition also comprises and choline. Preferably, these nutrients are provided in the amounts as detailed above, or elsewhere within this specification, for example in the tables below.

According to an embodiment, the composition of the invention further comprises one, a combination of several or all nutrients selected from the group of folic acid (vitamin B₉), thiamine (vitamin B₁) and zinc. Preferably, these nutrients are provided in the amounts as detailed above, or elsewhere within this specification, for example in the tables below.

According to an embodiment, the nutritional composition of the invention comprises macronutrients and soluble fiber in the quantities indicated in Table 1 below.

It is noted that in the tables and claims herein, quantities are indicated as percent per 100 g of complete dry matter. Powdered (substantially dry), reconstitutable formulas available in the market generally comprise a few grams of residual water, the amount of which varies and which is thus not considered within the indicated values.

**Table 1: Preferred amounts of macronutrients, key fatty acids and soluble fiber in the nutritional composition of the invention**

| **Nutrient** | | Unity | Weight per 100g |
|---|---|---|---|
| **Fat** | | g | 15-26 |
| | DHA | mg | 10-60 |
| | ARA | mg | 10-60 |
| | LA | g | 1-10 |
| | ALA | mg | 300-900 |
| **Protein** | | g | 11-20 |
| **Available carbohydrates** | | g | 40-65 |
| **Soluble fiber** | | g | at least 1 |

According to a preferred embodiment, the nutritional composition of the invention comprises macronutrients and soluble fiber in the quantities (percent per 100 g dry matter) indicated in Table 2 below:

**Table 2: More preferred amounts of macronutrients, key fatty acids and soluble fiber in the nutritional composition of the invention**

| **Nutrient** | | Unity | Weight per 100g |
|---|---|---|---|
| **Fat** | | g | 17-24 |
| | DHA | mg | 15-50 |
| | ARA | mg | 15-50 |
| | LA | mg | 2-7 |
| | ALA | mg | 400-800 |
| **Protein** | | g | 13-20 |
| **Available carbohydrates** | | g | 45-60 |
| **Solube fiber** | | g | at least 2 |

According to a most preferred embodiment, the nutritional composition of the invention comprises macronutrients and soluble fiber in the quantities (percent per 100 g dry matter) indicated in Table 3 below:

**Table 3: Most preferred amounts of macronutrients, key fatty acids and soluble fiber in the nutritional composition of the invention**

| **Nutrient** | | Unity | Weight per 100g |
|---|---|---|---|
| **Fat** | | g | 18-22 |
| | DHA | mg | 20-40 |
| | ARA | mg | 20-40 |
| | LA | mg | 3-6 |
| | ALA | mg | 500-700 |
| **Protein** | | g | 19-22.5 |
| **Available carbohydrates** | | g | 43-50 |
| **Soluble fiber** | | g | at least 3 |

With respect to the micronutrients, in particular vitamins and minerals, exemplary preferred, more preferred and most preferred quantities of key micro-nutrients for the purpose of the present invention are set out in Tables 4-6 below.

**Table 4: Preferred amounts of some key micronutrients in the nutritional composition of the invention**

| **Minerals and vitamins** | | Unity | Weight per 100g |
|---|---|---|---|
| | Iron | mg | 2-50 |
| | Choline | mg | 50-300 |
| | Vitamin B₉ (folic acid) | µg | 100-350 |
| | Vitamin B₁ (thiamine) | mg | 0.3-1.8 |
| | Zinc | mg | 5-50 |

**Table 5: More preferred amounts of some key micronutrients in the nutritional composition of the invention**

| **Minerals and vitamins** | | Unity | Weight per 100g |
|---|---|---|---|
| | Iron | mg | 4-30 |
| | Choline | mg | 80-250 |
| | Vitamin B₉ (folic acid) | µg | 120-300 |
| | Vitamin B₁ (thiamine) | mg | 0.4-1.6 |
| | Zinc | mg | 7-40 |

**Table 6: Most preferred amounts of some key micronutrients in the nutritional composition of the invention**

| **Minerals and vitamins** | | Unity | Weight per 100g |
|---|---|---|---|
| | Iron | mg | 6-15 |
| | Choline | mg | 110-190 |
| | Vitamin B₉ (folic acid) | µg | 130-270 |
| | Vitamin B₁ (thiamine) | mg | 0.5-1.4 |
| | Zinc | mg | 8-20 |

The Tables 1-3 and 4-6 concerning macro- and micronutrients, respectively, may be combined in any way in accordance with the nutritional composition of the present invention. Accordingly, a nutritional composition of the invention may be defined by combining Table 1 and Table 4, Table 1 and Table 5, Table 1 and Table 6. Further embodiments are defined by combining Table 2 with Table 4, Table 3 with Table 5 and Table 2 with Table 6. Further embodiments are defined by combining Table 3 with Table 4, Table 3 with Table 5 and Table 3 with Table 6. Accordingly, Tables 1-6 relate to 9 different embodiments according to the present invention.

The nutrients as listed in Tables 1-9 may be completed with nutrients as detailed further above in order to provide the nutritional composition of the invention.

In particular, the nutritional composition preferably comprises sphyngomyelin and sialic acid, which are not listed in the above tables, but which are preferably present and added by way of milk ingredients, such as whole milk, skimmed milk, and/or buttermilk, for example.

An embodiment of a nutritional composition according to the present invention is detailed in Table 7 below, wherein the low limit value and the high limit value define the range of the weight contribution in g of a respective nutrient or ingredient per 100 g of dry matter of the nutritional composition.

**Table 7: Embodiment of the nutritional composition of the invention**

| Nutrient | | Unity | Per 100 g dry matter | |
|---|---|---|---|---|
| | | | Low limit value | High limit value |
| **Total fat** | | g | **17.43** | **24.60** |
| | Milk fat | g | 5.04 | 7.11 |
| | Non-milk fat | g | 12.00 | 16.94 |
| | Lecithin | g | 0.30 | 0.60 |
| Fatty acids provided by the above fat ingredients: | | | | |
| | Linoleic acid | g | 3.08 | 6.15 |
| | Linolenic acid | mg | 445.88 | 891.76 |
| | DHA | mg | 26.91 | 53.81 |
| | ARA | mg | 26.14 | 52.28 |
| **Total protein** | | g | **14.08** | **20.00** |
| **Total available carbohydrates** | | g | **46.87** | **66.17** |
| **Total dietary Fibre** | | g | **3.04** | **4.29** |
| | Inulin and oligosaccharides | g | 3.04 | 4.29 |
| **Minerals (Ash)** | | g | **3.00** | **6.00** |
| | Calcium | mg | 760.61 | 1073.80 |
| **Micronutrients¹** | | | | |
| | Sphingomyelin² | mg | 25.63 | 102.50 |
| | Choline³ | mg | 68.68 | 274.70 |
| | Sialic acid² | mg | 49.71 | 198.85 |
| | Taurine | mg | 39.21 | 69.19 |
| | Iron | mg | 6.15 | 12.30 |
| | Zinc | mg | 9.99 | 19.99 |
| | Thiamin (B₁) | mg | 0.51 | 1.01 |
| | Niacin (vit B₃) | mg | 4.53 | 7.46 |
| | Pyridoxin (vit B₆) | mg | 0.44 | 0.67 |
| | Biotin (B₈) | µg | 3.66 | 5.60 |
| | Folic acid (vit B9) | µg | 103.78 | 207.56 |
| | Vitamin C | mg | 39.21 | 64.58 |
| | Vitamin A (retinol) | µg RE⁴ | 209.10 | 295.20 |
| | Vitamin D | µg CE⁵ | 5.23 | 10.46 |
| | Vitamin E | mg | 4.18 | 7.84 |
| | Vitamin K | µg | 14.35 | 30.75 |
| | Vitamin B₂ | mg | 0.79 | 1.69 |
| | Pantothenic acid | mg | 0.77 | 1.54 |
| **Total solids** | | g | **100 g** | |

| | | | | |
|---|---|---|---|---|
| 1) These micronutrients are included in the ash; 2) These nutrients are naturally provided, in particular by milk ingredients; 3) These nutrients are separately added and also naturally provided to a substantial extent; 4) Retinol equivalents; 5) Calciferol equivalents. | | | | |

Another embodiment of a nutritional composition according to the present invention is detailed in Table 8 below:

**Table 8: Preferred embodiment of the nutritional composition of the invention**

| Nutrient | | Unity | Per 100 g dry matter | |
|---|---|---|---|---|
| | | | Low limit value | High limit value |
| **Total fat** | | g | **18.45** | **22.55** |
| | Milk fat | g | 5.33 | 6.52 |
| | Non-milk fat | g | 12.70 | 15.53 |
| | Lecithin | g | 0.3 | 0.55 |
| Fatty acids provided by the above fat ingredients: | | | | |
| | Linoleic acid | g | 3.69 | 4.51 |
| | Linolenic acid | mg | 535.06 | 653.96 |
| | DHA | mg | 32.29 | 39.46 |
| | ARA | mg | 31.37 | 38.34 |
| **Total protein** | | g | **14.91** | **18.22** |
| **Total available carbohydrates** | | g | **49.63** | **60.66** |
| **Total dietary Fibre** | | g | **3.22** | **3.94** |
| | Inulin and oligosaccharides | g | 3.22 | 3.94 |
| **Minerals (Ash)** | | g | 3.79 | 4.63 |
| | Calcium | mg | 805.35 | 984.32 |
| **Micronutrients¹** | | | | |
| | Sphingomyelin² | mg | 35.88 | 76.88 |
| | Choline³ | mg | 96.15 | 206.03 |
| | Sialic acid² | mg | 69.60 | 149.14 |
| | Taurine | mg | 41.51 | 59.96 |
| | Iron | mg | 6.97 | 10.66 |
| | Zinc | mg | 11.33 | 17.32 |
| | Thiamin (vit. B₁) | mg | 0.58 | 0.88 |
| | Niacin (vit. B₃) | mg | 4.80 | 6.93 |
| | Pyridoxin (vit. B₆) | mg | 0.44 | 0.67 |
| | Biotin (vit. B₈) | µg | 3.66 | 5.17 |
| | Folic acid (vit. B₉) | µg | 117.62 | 179.89 |
| | Vitamin C | mg | 41.51 | 59.96 |
| | Vitamin A | µg RE⁴ | 209.10 | 319.80 |
| | Vitamin D | µg CE⁵ | 5.92 | 9.06 |
| | Vitamin E | mg | 4.44 | 6.80 |
| | Vitamin K | µg | 17.43 | 26.65 |
| | Vitamin B₂ | mg | 0.96 | 1.47 |
| | Pantothenic acid | mg | 0.87 | 1.54 |
| **Total solids** | | g | **100** | |

The small numbers 1-5 have the same meaning as in Table 7.

The nutritional composition as defined in Tables 1-8 generally also comprises iodine. It is also naturally provided by milk ingredients and is generally present at the amounts indicated above.

In any event, the nutritional compositions of the invention as described in the tables above preferably comprise prebiotics and/or probiotics as defined further above, more preferably further nutrients as detailed in the present specification.

The nutritional composition of the present invention may be provided as a ready-to-drink liquid form or in the form of a dry powder to be reconstituted with water, or another water or milk based liquid. Preferably, the nutritional composition is provided in powdered form, and reconstituted by addition of water by the consumer shortly (for example 1 minute to 2 hours) before consumption. As is usual, the water has to be clean, that is preferably bottled water or has been boiled and filtered and cooled to 60°C or lower before addition to the nutritional composition.

If the nutritional composition is provided in the form of a powder, it is preferably reconstituted by mixing 12-20 g powder in 1 dl water, preferably 14-20 g powder, more preferably about 16-18 g powder per 1 dl (0.1 litre) water.

The typical serving size is about 225 ml, so that for example 36 g of powder are added to 225 ml water. Per day, ideally 2 to 3 serving sizes are consumed per child of 3 to 6 years.

The nutritional composition preferably provides 400-500 kcal, more preferably 420-480 kcal per 100 g of dry weight of the composition. According to an embodiment, per dl of the reconstituted formula, the composition provides preferably 40-80, more preferably 45-75, most preferably 50-70 kcal. The caloric content of the nutritional composition per volume (for example 100 ml) may generally also be determined on the basis of the caloric content per 100 g and considering reconstitution quantities as indicated above.

According to a preferred embodiment, the nutritional composition is a growing up milk, also referred to as a GUM. More preferably, the nutritional composition is a powdered GUM, for example a GUM that is substantially free of starch.

According to an embodiment, the nutritional composition is adapted to children of an age of 3-7, preferably 3-6, more preferably 3-5, and most preferably 3-4 years.

The nutritional composition provides several benefits associated with the healthy mental, behavioural and intellectual development of the child.

In an aspect and/or embodiment, the nutritional composition is used to provide nutrients that have at least one of the following purposes: nutrients that (1) support the structural development of the brain, (2) are needed for the biochemical processes taking place in the brain, and/or (3) that provide energy necessary for the functioning of the brain. Preferably, the nutritional composition provides nutrients for (1), for (1) and (2), for (1) and (3), for (2) and (3) or for (1), (2) and (3) above.

In an aspect and/or embodiment, the nutritional composition supports and/or improves overall cognitive performance, for example cognitive capacities and/or abilities in a child, as well as intelligence. Specific aspects and/or embodiment of cognitive performance are discussed in further detail below.

In an aspect and/or embodiment, the nutritional composition of the invention contributes to and/or improves learning and/or memory. In particular, the nutritional composition contributes to and/or improves one, a combination of two or more or all of: long term memory, short term memory, working memory, verbal memory, auditory memory, spatial memory, visual memory, recognition memory and recall.

In an aspect and/or embodiment, the nutritional composition of the invention contributes to and/or improves attention. In particular, the nutritional composition contributes to and/or improves one, a combination of two or more or all of: concentration, alertness, maintenance of focus. The nutritional composition preferably prevents distraction, for example at school and reduces impulsiveness. This aspect of cognitive performance includes one or more of selective attention, sustained attention, divided attention and the switching of attention.

In an aspect and/or embodiment, the nutritional composition of the invention contributes to and/or improves executive functioning. In particular, the nutritional composition contributes to and/or improves one, a combination of two or more or all of: mental flexibility, conceptual thinking, abstract reasoning, and problem solving. Preferably, the nutritional composition contributes to and/or improves the formation and maintenance of plans, rule learning, mental set switching and concept formation.

In an aspect and/or embodiment, the nutritional composition of the invention contributes to and/or improves psychomotor and/or perceptual performance. In particular, the nutritional composition contributes to and/or improves one, a combination of two or more or all of: motor coordination, dexterity, spatial skills. Preferably, the nutritional composition improves and/or contributes to one or more of: psycho-motor integration, perceptual and motor tasks, spatial ability and the like.

In an aspect and/or embodiment, the nutritional composition of the invention contributes to and/or improves language skills. In particular, the nutritional composition contributes to and/or improves one, a combination of two or more or all of: verbal ability, language understanding, verbal comprehension, verbal learning.

In an aspect and/or embodiment, the nutritional composition of the invention contributes to and/or improves mood and/or the ability to interact with the environment. Accordingly, the nutritional composition contributes to and/or improves one, a combination of two or more or all of: reduction of anxiety, reduction of excitability, curiosity, the tendency to explore the environment confidently, contentedness, interaction with others, and reduction of fussiness.

In an aspect and/or embodiment, the nutritional composition supports the healthy, normal or improved behavioural, cognitive, communicational, such as language, expressive and receptive communication, motor and social-emotional development of a child.

According to a preferred embodiment, the composition of the present invention contributes to the healthy development and/or improved development in a child of one, a combination of two or more, or all selected from: memory, learning capacity, comprehension, alertness, attention, concentration capacity, conceptual thinking, abstract thinking, verbal abilities, language comprehension, processing speed, curiosity, and confident interaction with the environment.

Cognitive performance of children of 2.5 to about 7 years may be assessed, for example, with the WPPSI-III test (Wechsler Preschool and Primary Scale of Intelligence, published in 2002 for children of the age of 2 years 6 months to 7 years, three months). This test can be commercially obtained, for example at:
http://pearsonassess.com/HAIWEB/Cultures/en-us/Productdetail.htm?Pid=015-8989-317&Mode=summary.

The WPPSI-III test contains several subtests for assessing different aspects of a child's cognitive functioning and/or intelligence. There are subtests test for measuring verbal comprehension and language skills, intelligence, and mental and/or cognitive processing speed.

The verbal subtests of the WPPSI-III comprise tests for assessing the child's:
- ability to handle information, where a child must either point to a picture or verbally answer brief oral questions about commonplace objects and events.
- comprehension, where a child verbally responds to questions of consequences of events.
- vocabulary, where a child names pictured items and provides verbal definitions of words.
- ability to recognize similarities, where a child completes a sentence that contains a verbal analogy.
- ability to name a picture, where the child names pictures that are displayed singularly in a booklet.
- receptive vocabulary. The child looks at a group of four pictures and points to the one that an examiner describes aloud.
- word reasoning. The child is read an increasingly specific series of one to three clues and identifies the common object or concept being described.

The performance subtests of the WPPSI-III comprise tests for assessing the child's:
- ability to assemble objects. The child is required to fit puzzle pieces together to form a meaningful whole.
- ability of block design. The child reproduces patterns made from a 1- or 2-colored block.
- ability to mentally complete a picture. The child identifies what is missing from pictures of common objects.
- matrix reasoning. The child looks at an incomplete matrix and selects the missing section from four or five response options.
- identify picture concepts. The child is presented with two or three rows of pictures and chooses one picture from each row to form a group with a common organizational concept.
- search for symbols. The child indicates, by marking a box, whether a target symbol appears in a series of symbols.
- coding. Using a key, the child draws symbols that are paired with simple geometric shapes.
- In some or all of the subtest tasks, time may be measured to assess the child's performance.

As mentioned above, the nutritional composition of the invention supports and/or improves memory, in particular short term memory, working memory and/or long term memory, preferably at least short term memory and/or working memory. Short term memory and working memory can be more readily tested in typical experimental settings.

"Memory", for the purpose of the present specification, is an organism's mental ability selected from one, two (for example, (a) and (c)) or all three of (a) to store, (b) retain and (c) recall information. Memory phenomena that can be examined for the purpose of the present specification include: (1) knowledge (what is remembered?); (2) comprehension (what does it mean?); (3) context/function (why remember?); and (4) strategy (how to remember?). Memory is a complex psychological process that is not independent of a single memory domain process. Memory is related to several other cognition domains including, sensory memory, audio memory and visual memory. The terms of "memory" include:
"Short-term memory": a system for temporarily storing simple information, for example not more than 60 seconds, up to 30, up to 20 or up to 15 seconds, storing and managing information required to carry out cognitive tasks such as learning, reasoning, and comprehension, for example.
"Working memory": refers to short-term memory and requires the simultaneous storage and processing of information.
"Long-term memory" is memory that can last beyond a few, for example beyond 2-3, minutes to a few decades. Items of information stored as long-term memory may be available for a lifetime.

Memory is generally understood to be a process by which what is learned persists across time. In this sense, learning and memory are inextricably connected. Accordingly, the composition of the present invention supports and/or improves a child's learning and/or memory capacities.

For example, the nutritional composition of the present invention supports and/or increases a child's capacity recall words, numbers, pictures, symbols, letters, and/or tonal sequences within the short-term.

According to an aspect, the nutritional composition supports and/or improves at least one selected from learning capacity, alertness, attentiveness, and concentration capacity of a child.

According to an aspect, the composition of the present invention supports and/or improves a child's skills and/or abilities in one or more of (1) the comprehension and use of language, (2) mathematical abilities, (3) the conceptualization and reasoning.

The following examples are illustrative of some of the products and methods of making the same falling within the scope of the present invention. They are not to be considered in any way limitative of the invention. Changes and modifications can be made with respect to the invention. That is, the skilled person will recognize many variations in these examples to cover a wide area of formulas, ingredients, processing and mixtures to rationally adjust the nutrients and other elements of the invention for a variety of applications.

### Examples

### Example 1: Nutritional composition according to the invention

A powdered milk for children of 3 to 6 years is produced by preparing a slurry of ingredients, in particular skimmed milk powder, whole milk, buttermilk, maltodextrin, sugar, lactose, vegetable oil, flavouring ingredients, an emulsifier, a vitamin mix, minerala and trace elements mixes, prebiotics, and an oil comprising DHA and ARA. The slurry is subjected to heating for evaporation and then transformed to a powder by spray-drying. Thereafter, two different probiotic microorganisms, *Bifidobacterium longum* (ATCC BAA-999) and *Lactobacillus paracasei* (CNCM I-2116) in powdered form are added so as to each provide at least 1x10⁶ CFU per g of powder. The final composition of the nutrients of the growing up milk per 100 grams of dry matter (residual water not considered) is provided in Table 9 below. The exemplary composition of Table 9 contains about 455 kcal per 100 g dry matter.

**Table 9: Nutrients of the nutritional composition of the invention**

| Nutrient | | Unity | Per 100 g dry matter in unity |
|---|---|---|---|
| **Total fat** | | g | **20.50** |
| | Milk fat | g | 5.92 |
| | Non-milk fat | g | 14.11 |
| | Lecithin | g | 0.46 |
| Fatty acids provided by the above fat ingredients: | | | |
| | Linoleic acid | g | 4.10 |
| | α-linolenic acid | mg | 594.51 |
| | DHA | mg | 35.88 |
| | ARA | mg | 34.85 |
| **Total protein** | | g | **16.56** |
| | Casein | g | 12.75 |
| | Whey protein | g | 3.81 |
| **Total available carbohydrates** | | g | **55.15** |
| | Lactose | g | 30.99 |
| | Saccharose | g | 7.83 |
| | Maltodextrin | g | 15.59 |
| **Total dietary Fibre** | | g | **3.58** |
| | Inulin and oligosaccharides | g | 3.58 |
| **Minerals (Ash)** | | g | **4.21** |
| | Sodium | mg | 252.15 |
| | Potassium | mg | 823.08 |
| | Chloride | mg | 591.43 |
| | Calcium | mg | 894.83 |
| | Phosphorus | mg | 509.43 |
| | Magnesium | mg | 58.43 |
| | Manganese | µg | 60.48 |
| | Selenium | µg | 5.74 |
| **Micronutrients¹** | | | |
| | Sphingomyelin² | mg | 51.25 |
| | Choline³ | mg | 137.35 |
| | Sialic acid² | mg | 99.43 |
| | Taurine | mg | 46.13 |
| | Iodine² | µg | 84.05 |
| | Iron | mg | 8.20 |
| | Zinc | mg | 13.33 |
| | Thiamin (B₁₎ | mg | 0.68 |
| | Niacin (vit B₃) | mg | 5.33 |
| | Pyridoxin (vit B₆) | mg | 0.51 |
| | Biotin (B₈) | µg | 4.31 |
| | Folic acid (vit B9) | µg | 138.38 |
| | Vitamin C | mg | 46.13 |
| | Vitamin A (retinol) | µg RE⁴ | 246.00 |
| | Vitamin D | µg CE⁵ | 6.97 |
| | Vitamin E | mg | 5.23 |
| | Vitamin K | µg | 20.50 |
| | Vitamin B₂ | mg | 1.13 |
| | Pantothenic acid | mg | 1.03 |
| **Total dry matter** | | g | 100 |

The small numbers 1-5 have the same meaning as in Table 7 above.

### Example 2: Clinical Study for Assessing General Cognitive Performance and Intelligence of Children of 3 to 6 Years of Age

In a clinical study, the cognitive skills of children are to be assessed.

For the study, a total of 200 healthy children of the age of 3 to 5 years of both sexes are recruited. The children are split in two groups. One group receives the growing up milk as detailed in Example 1, the other group is the control group and receives (full cream) milk powder. Both groups will consume habitual food, in particular food as usual. The children receiving the nutritional composition of the invention receive 2 serving sizes per day. One serving size is obtained by mixing 36 g of the powder with 210 ml water. The children of the control group receive an equivalent amount of the control powder.

Following a test period of one year, the children's healthy development in the general areas of general cognitive abilities and general intelligence is measured. In particular, it is assessed if the children have any cognitive benefits. For assessment, the Columbia Mental Maturity Scale, of Bessie B. Burgemeister, Lucille Hollander Blum, Irving Lorge, published by Harcourt Brace Janovich, Inc., 1972, is used. This test can be commercially obtained at:
http://pearsonassess.com/HAIWEB/Cultures/en-us/Productdetail.htm?Pid=015-8035-917&Mode=summary

The test entails presenting the children with a series of cards (3-5 cards) presented at a time and the child has to select the odd one out of the group - what does not fit in that category. The test thus measures how children associate different meanings together and can understand the formation of a category.

The test is adapted to the child's culture and language. According to the protocol of this test, each child's capacity is assessed separately.

At the end of the study, the comparison between the groups shows that the group receiving the nutritional composition performs better in the areas assessed by the test. In particular, in the group receiving the nutritional composition of the present invention, more children will benefit cognitively.

The results indicate improved performance on at least one measure pertaining to attention, learning, comprehension, conceptual thinking, memory, alertness, concentration and mood in children receiving the composition of the present invention.

These results show that the nutritional composition of the invention is a growing up milk that supports and/or improves the cognitive performance, in particular memory and concentration capacities in children of the age of 3 to 5 years.

### Example 3: Clinical Study for Assessing Specific Aspects of Cognitive Performance of Children of 3 to 5 Years of Age

This study follows the same general protocol as in Example 2, with the difference that selected aspects of the children's intelligence are assessed using the WPPSI-III test (Wechsler Preschool and Primary Scale of Intelligence, published in 2002 for children of the age of 2 years 6 months to 7 years, three months). Details of this test are provided above.

In this test, verbal intelligence, performance intelligence, full scale IQ, processing speed and general language skills are differentiated. Different aspects of this test are detailed in the present specification above.

Similar to the result obtained in Example 2, the children receiving the nutritional composition of the present invention have better results in various areas of measurement of the test, in particular in the areas of attention, executive functions such as forming picture concepts, reasoning, receptive vocabulary, word reasoning, processing speed, and memory.

These results show that the nutritional composition of the invention is a growing up milk that supports and/or improves the cognitive performance as defined in the present specification, in particular the learning and memory capacities in children of the age of 3 to 5 years. More specifically, the nutritional composition of the invention is a growing up milk that supports/improves attention capacities in children of the age of 3 to 5 years.

## Claims

1. The use of a nutritional composition for one or more selected from supporting, sustaining and improving cognitive performance in a child of 3 to 6 years, said nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

2. The use of claim 1, wherein the child is a child of 3-5 years.

3. The use of claim 1 or 2, wherein cognitive performance refers to one, a combination of two or more, or all selected from: memory, learning capacity, comprehension, alertness, attention, concentration capacity, conceptual thinking, abstract thinking, verbal abilities, language comprehension, processing speed, curiosity, and confident interaction with the environment.

4. The use of any one of the preceding claims, wherein said nutritional formula comprises DHA and/or ARA.

5. The use of any one of the preceding claims, wherein the nutritional formula further comprises linoleic acid and/or α-linolenic acid.

6. The use of any one of the preceding claims, wherein said nutritional composition comprises, per 100 gram of dry matter, at least 2 mg (milligram) iron.

7. The use of any one of the preceding claims, wherein said nutritional composition comprises, per 100 gram of dry matter, at least 20 mg iodine.

8. The use of any one of the preceding claims, wherein said nutritional composition comprises, per 100 gram of dry matter, at least 20 mg sphingomyelin.

9. The use of any one of the preceding claims, wherein the nutritional composition comprises, per 100 gram of dry matter, at least 50 mg sialic acid.

10. The nutritional composition, wherein the nutritional composition comprises, per 100 gram of dry matter, at least 30 mg choline.

11. The use of any one of the preceding claims, wherein the nutritional composition comprises one, a combination of several or all selected of the group of DHA, ARA, LA, ALA, iodine, and iron.

12. The use of any one of the preceding claims, wherein the nutritional composition further comprises one, a combination of several or all selected of sphingomyelin, sialic acid, and choline.

13. The use of any one of the preceding claims, wherein the nutritional composition further comprises one, a combination of several or all selected of folic acid (vitamin B₉), thiamine (vitamin B₁), and zinc.

14. The use of any one of the preceding claims, wherein said protein source provides about 10 to about 22 percent of the total energy, said source of available carbohydrates provides from about 30 to about 60 percent of the total energy, and said lipid source provides from about 30 to about 55 percent of the total energy of the composition.

15. The use of any one of the preceding claims, wherein said nutritional composition comprises at least two different probiotic microorganisms, each of said microorganisms being provided at about 1 x 10⁵ to 1x 10⁹ CFU per g of dry matter of the composition.

16. The use of any one of the preceding claims, wherein said prebiotic comprises one or more selected from inulin, fructooligosaccharides, or a mixture of inulin and fructooligosaccharides.

17. The use of any one of the preceding claims, wherein said nutritional composition is a growing-up milk, which is provided in powdered form.

18. The use of any one of the preceding claims, wherein said nutritional composition comprises the nutrients per 100 g of dry matter as detailed in the table below:
| Nutrient | | Unity | Per 100 g dry matter | |
|---|---|---|---|---|
| | | | Low limit value | High limit value |
| **Total fat** | | g | **17.43** | **24.60** |
| | Milk fat | g | 5.04 | 7.11 |
| | Non-milk fat | g | 12.00 | 16.94 |
| | Lecithin | g | 0.30 | 0.60 |
| Fatty acids provided by the above fat ingredients: | | | | |
| | Linoleic acid | g | 3.08 | 6.15 |
| | Linolenic acid | mg | 445.88 | 891.76 |
| | DHA | mg | 26.91 | 53.81 |
| | ARA | mg | 26.14 | 52.28 |
| **Total protein** | | g | **14.08** | **20.00** |
| **Total available carbohydrates** | | g | **46.87** | **66.17** |
| **Total dietary Fibre** | | g | **3.04** | **4.29** |
| | Inulin and oligosaccharides | g | 3.04 | 4.29 |
| **Minerals (Ash)** | | g | **3.00** | **6.00** |
| | Calcium | mg | 760.61 | 1073.80 |
| **Micronutrients¹** | | | | |
| | Sphingomyelin² | mg | 25.63 | 102.50 |
| | Choline³ | mg | 68.68 | 274.70 |
| | Sialic acid² | mg | 49.71 | 198.85 |
| | Taurine | mg | 39.21 | 69.19 |
| | Iron | mg | 6.15 | 12.30 |
| | Zinc | mg | 9.99 | 19.99 |
| | Thiamin (B₁) | mg | 0.51 | 1.01 |
| | Niacin (vit B₃) | mg | 4.53 | 7.46 |
| | Pyridoxin (vit B₆) | mg | 0.44 | 0.67 |
| | Biotin (B₈) | µg | 3.66 | 5.60 |
| | Folic acid (vit B9) | µg | 103.78 | 207.56 |
| | Vitamin C | mg | 39.21 | 64.58 |
| | Vitamin A (retinol) | µg RE⁴ | 209.10 | 295.20 |
| | Vitamin D | µg CE⁵ | 5.23 | 10.46 |
| | Vitamin E | mg | 4.18 | 7.84 |
| | Vitamin K | µg | 14.35 | 30.75 |
| | Vitamin B₂ | mg | 0.79 | 1.69 |
| | Pantothenic acid | mg | 0.77 | 1.54 |
| **Total solids** | | g | **100 g** | |

19. A method for supporting and/or improving cognitive performance in a child of 3 to 6 years, said method comprising the step of administrating a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

20. A method of providing nutrition, the method comprising the step of administering to a child of 3 to 6 years a nutritional composition comprising:
- one or more protein source,
- one or more source of available carbohydrates,
- one or more lipid source,
- one or more probiotic microorganism, and,
- prebiotics,
wherein said lipid source comprises DHA (docosahexaenoic acid).

21. A method for preparing a nutritional composition useful for supporting and/or improving brain function, said method comprising mixing:
- one or more protein source,
- one or more source of available carbohydrates,
- one or more lipid source,
- one or more probiotic microorganism, and,
- prebiotics,
wherein said lipid source comprises DHA (docosahexaenoic acid), and thereby obtaining the nutritional composition of the invention.

22. The uses and methods according to the preceding claims, wherein said nutritional composition is a growing up milk.

23. The uses and methods according to the preceding claims, wherein said nutritional composition is provided in powdered, reconstitutable form.
